**Europäisches Patentamt**

**European Patent Office**

**Office européen des brevets**

(19)

(11) Veröffentlichungsnummer: **0 373 585**
**A1**

(12) # EUROPÄISCHE PATENTANMELDUNG

(21) Anmeldenummer: **89122907.2**

(22) Anmeldetag: **12.12.89**

(51) Int. Cl.5: **A61B 5/085, G01R 27/00**

(30) Priorität: **13.12.88 DE 3841932**
**09.02.89 DE 3903857**

(43) Veröffentlichungstag der Anmeldung:
**20.06.90 Patentblatt 90/25**

(84) Benannte Vertragsstaaten:
**AT BE CH DE ES FR GB GR IT LI LU NL SE**

(71) Anmelder: **Schumann, Klaus, Prof. Dr. med.**
**Franz-Wiedemeier-Strasse 27**
**D-7900 Ulm(DE)**

Anmelder: **Walliser, Dieter, Dr.**
**Gässele 5**
**D-7913 Senden-Witzighausen(DE)**

(72) Erfinder: **Schumann, Klaus, Prof. Dr. med.**
**Franz-Wiedemeier-Strasse 27**
**D-7900 Ulm(DE)**
Erfinder: **Walliser, Dieter, Dr.**
**Gässele 5**
**D-7913 Senden-Witzighausen(DE)**

(74) Vertreter: **Zinnecker, Armin, Dipl.-Ing. et al**
**Rechtsanwälte Eduard Lorenz - Dipl.-Ing.**
**Hans-K. Gossel Dr. Ina Philipps - Dr. Paul B.**
**Schäuble Dr. Siegfried Jackermeier Dipl.-Ing.**
**Armin Zinnecker**
**Widenmayerstrasse 23 D-8000 München**
**22(DE)**

(54) **Verbesserte Bestimmung des Atemwegswiderstandes nach der Oszillationsmethode.**

(57) Eine Vorrichtung (8) zur Bestimmung der Atemwegsimpedanz nach der Oszillations-methode besteht aus einer Pumpe (1) zum Erzeugen einer Luftschwingung mit einer vorbestimmten Amplitude und Frequenz, einem Luftschlauch-Mundstück (2) für den Patienten (3), einem Verbindungsschlauch (4) von der Pumpe (1) zum Mundstück (2), einem geeichten Luftschlauch (5) mit einer vorbestimmten Referenzimpedanz ($Z_{REF}$) vom Mundstück (2) ins Freie (6), einem Meßschlauch (7) vom Mundstück (2) zur Vorrichtung (8), einem Druckaufnehmer (9) zum Messen des Luftdrucks im Meßschlauch (7) und zum Erzeugen eines analogen Spannungssignals (y), welches ein Maß ist für die Impedanz ($Z_{GES}$) des Gesamtsystems. Ein Phasenmeßglied (10) bestimmt die Phasenverschiebung zwischen dem Volumenstrom dV/dt und dem gemessenen Wechseldruck dP. Mittels einer Lichtschranke wird die Stellung der Pumpe und somit die Phase von dV/dt abgetastet, die mit der Phase von dP verglichen wird. Das Phasenmeßglied liefert ein dem Gesamtphasenwinkel analoges Signal, vorzugsweise ein analoges Spannungssignal (x). Mittels einer Recheneinrichtung können die Atemwegsimpe-danz ($Z_{AW}$, der Realteil der Atemwegsimpedanz, der Phasenwinkel der Atemwegsimpedanz sowie der Phasen-winkel der Gesamtimpedanz ($Z_{GES}$) bestimmt werden.

EP 0 373 585 A1

# Fig.1

EP 0 373 585 A1

## Verbesserte Bestimmung des Atemwegswiderstandes nach der Oszillationsmethode

Die Erfindung betrifft eine Vorrichtung zur Bestimmung des Atemwegswiderstandes eines Patienten nach der Methode der oszillatorischen Widerstandsbestimmung nach dem Oberbegriff des Anspruchs 1. Eine derartige Vorrichtung ist in der Praxis bekannt. Vorzugsweise handelt es sich bei dieser Vorrichtung um das Gerät "Siregnost FD 5" der Firma Siemens.

Die Hauptfunktion der Atemwege besteht im ungestörten Gasaustausch zwischen Außenwelt und Lungenparenchym. Störungen lassen sich am sichersten mit objektiven Atemwegswiderstandsmessungen beurteilen. Hierzu wurden in den vergangenen 30 Jahren Atemwegswiderstandsmeßgeräte entwickelt, die auf den unterschiedlichsten Prinzipien beruhen, wie die Ganzkörperplethysmographie, die Oszillationsmethode, die Wechseldruckmethode und die Verschlußdruckmethode.

Beim Atmen muß die Muskulatur visköse und elastische Widerstände überwinden. Der visköse Atemwiderstand wird durch den Strömungswiderstand der Atemwege (Resistance) sowie durch die Gewebereibung von Lunge und "Thorax" hervorgerufen. Der Strömungswiderstand der Atemwege (Resistance) gilt als der pathophysiologisch wichtigste Parameter der Atemmechanik. Der Strömungswiderstand R der Atemwege (Resistance) ist definiert als Quotient der Druckdifferenz dp zwischen Alveolar- und Munddruck sowie des damit erzeugten Volumenstroms dV/dt.

Zwei bekannte Methoden zur Bestimmung des Atemwegswiderstandes sind die Ganzkörperplethysmographie und die oszillatorische Bestimmung des Atemwegswiderstandes. Die Ganzkörperplethysmographie ist als Standard- oder Referenzmethode akzeptiert.

Bei der Messung nach der Methode der Ganzkörperplethysmographie sitzt der Patient in einer starren, luftdicht abgeschlossenen Kammer mit bekanntem Volumen. Über ein nach außen führendes Atemrohr ventiliert er bei zugeklemmter Nase auf BTPS-Bedingungen angefeuchtete und erwärmte Luft. Der Volumenstrom und der Kammerdruck werden gemessen und auf einen X-Y-Schreiber gegeneinander aufgetragen. Zur Berechnung des Atemwegswiderstandes ist darüber hinaus die Bestimmung der atmosphärisch-alveolären Druckdifferenz erforderlich, die indirekt erfolgt. Dazu verschließt man das Atemrohr durch ein elektromagnetisches Ventil ("Shutter") luftdicht. Wenn der Patient jetzt zu atmen versucht, erzeugt er in der Kammer und in der Lunge einander entgegengesetzte Druckschwankungen, die zueinander in einem bestimmten Verhältnis stehen. Im Atemsystem herrscht dabei von den Alveolen bis zu Mund und Nase derselbe Druck, d.h. der proximal des Shutters gemessene Druck entspricht dem jeweiligen Alveolardruck. Alveolar- und Kammerdruck werden synchron gemessen und wieder auf den X-Y-Schreiber gegeneinander aufgezeichnet. Durch Division der Steigungen der Kammerdruck-Alveolardruckkurve und der Kammerdruck-Volumenstromkurve läßt sich dann der Atemwegswiderstand (Resistance) bestimmen.

Bei der Anwendung der Oszillationsmethode zur Bestimmung des Atemwegswiderstandes werden dem Atemtrakt des Patienten über den Mund mit Hilfe einer ventillosen Pumpe Oszillationen von beispielsweise 10 Hz aufgezwungen. Die Interpretation der Ergebnisse erfolgt über ein mechano-akustisches Modell des Atemtraktes, dessen Widerstandsverhalten durch einen analogen Wechselstromkreis mit parallel- und in Serie geschalteten realen, kapazitiven und induktiven Widerständen beschrieben wird.

Besonders geeignet zur Bestimmung des Atemwegswiderstandes nach der Methode der oszillatorischen Widerstandsbestimmung ist das Gerät "Siregnost FD 5" der Firma Siemens. Bei der oszillatorischen Atemwegswiderstandsbestimmung mit diesem Gerät wird der menschlichen Atemfrequenz von 0,2 - 0,3 Hz ein höherfrequenter Oszillationsstrom von 10 Hz aufgelagert. Er wird durch eine ventillose Membranpumpe mit einem Hubvolumen von 1,4 ml erzeugt. Es wird dabei nicht nur der Atemtrakt, sondern auch ein am Mundstück angeschlossener Plastikschlauch mit bekanntem Radius und bekannter Länge oszilliert. Der Schlauch dient als Referenz-Widerstand im Sinne einer Impedanz, dessen Wert von 10 mbar/(l x s) überwiegend durch die Induktivität der oszillierten Luftsäule verursacht wird. Vom Hersteller wurde bei einer stationären Strömung von 0,5 l/s ein Strömungswiderstand von 1,5 mbar/(lxs) im Referenzschlauch gemessen. Über den Schlauch kann der Patient fast ungehindert atmen, sein Volumen von 85 ml vergrößert den Totraum nur unwesentlich. Der oszillierende Volumenstrom erzeugt im Mund einen Wechseldruck, der durch ein Mikrophon aufgenommen wird, das nach Filterung, Gleichrichtung und Glättung ein analoges Spannungssignal liefert.

Die sich daraus ergebende oszillatorische Resistance "Ros" kann am "Siregnost FD 5" direkt an einem Zeigerinstrument abgelesen werden. Da die Zeitkonstante am Gerät umschaltbar ist, lassen sich atemsynchrone Änderungen von "Ros" wahlweise unterdrücken oder am Zeigerausschlag sichtbar machen.

Die Messung von "Ros" ist auch für den Ungeübten einfach und rasch durchführbar. Zur Kalibrierung wird der Meßkopf mit einem Kalibrierstopfen oder der flachen Hand verschlossen.

2

Mit Hilfe eines Regelknopfes wird dann der Zeiger auf die Kalibriermarke eingestellt. Für die Messung muß die Nase des Probanden durch eine Nasenklemme abgedichtet werden.

Er nimmt nun das auf den Meßkopf gesetzte mitgelieferte Mundstück zwischen die Zähne und atmet bei fixierten Wangen ruhig ein und aus, ohne die Stimmritze willkürlich oder unbewußt zu verengen, bzw. zu verschließen. Nach drei bis vier Atemzyklen wird "Ros" auf der Zeigerskala des Gerätes direkt abgelesen. Im Atemzyklus kommt es zu Schwankungen von "Ros", die durch einen Wahlschalter gedämpft werden können. Die fortlaufende Aufzeichnung von "Ros" kann mit Hilfe eines EKG-Düsenschreibers erfolgen. Damit läßt sich die Änderung von "Ros" kontinuierlich über einen bestimmten Zeitraum registrieren.

Während die Meßgenauigkeit der Ganzkörperplethysmographie nicht angezweifelt wird, bestehen erhebliche Bedenken hinsichtlich der Aussagekraft der oszillatorischen Resistance "Ros".
Die Körperplethysmographie ist aufgrund des apparativen Aufwandes und der technisch schwierigen Handhabung ungeeignet für den Routinebetrieb bei Narkosen und bei der Intensivüberwachung und erst recht in der täglichen Praxis zur Behandlung der 3 Mill. Patienten in der Bundesrepublik Deutschland mit obstruktiven Atemwegserkrankungen; hierdurch werden volkswirtschaftliche Kosten in Höhe von schätzungsweise 20 Milliarden DM pro Jahr in der BRD hervorgerufen. Ersatzweise angeboten werden die Oszillationsmethode, Wechseldruckmethode und Verschlußdruckmethode. Aufgrund großer Streubreiten und unsicherer Meßergebnisse haben diese Verfahren in der Notfallmedizin überhaupt keinen und in der Praxis nur vereinzelt Eingang gefunden. Die oszillatorische Methode ist sehr handlich, also mit relativ geringem Aufwand durchführbar. Darüber hinaus erfordert sie keine Mitarbeit des Patienten und beeinträchtigt ihn nicht. Sie konnte sich jedoch aufgrund der bisherigen Mängel in der Praxis nicht durchsetzen.

Aufgabe der Erfindung ist es daher, eine verbesserte Vorrichtung zur Bestimmung des Atemwegswiderstandes eines Patienten nach der Methode der oszillatorischen Widerstandsbestimmung nach dem Oberbegriff des Anspruchs 1 zu schaffen, mit der eine genaue, repro duzierbare und aussagekräftige Bestimmung des Atemwegswiderstandes nach der Methode der oszillatorischen Widerstandsbestimmung möglich ist. Die Vorrichtung soll einfach aufgebaut sein, einfach zu handhaben sein und genaue Ergebnisse liefern, so daß sie auch in der Notfallmedizin und in der Praxis verwendbar ist.

Erfindungsgemäß wird diese Aufgabe durch die im kennzeichnenden Teil des Anspruchs 1 angegebenen Merkmale gelöst. Es ist eine Recheneinrichtung vorhanden, die geeignet ist, die Atemtraktimpedanz aus der Referenzimpedanz und der Gesamtimpedanz sowie dem Gesamtphasenwinkel zu errechnen. Durch Anwendung der Rechenvorschriften für komplexe Zahlen kann der Realteil und Imaginärteil der Atemtraktimpedanz bestimmt werden. Die Referenzimpedanz ist durch den geeichten Luftschlauch vorgegeben, dessen Impedanz und Phasenwinkel bekannt sind. Die Gesamtimpedanz kann durch den Druckaufnehmer gemessen werden; da der Volumenstrom dV/dt konstant bleibt, ist der gemessene Wechseldruck·am Mund ein Maß für die Gesamtimpedanz. Die Recheneinrichtung berechnet die Atemtraktimpedanz sowie den Realteil der Atemtraktimpedanz und den Atemtraktphasenwinkel aus der vorgegebenen Referenzimpedanz und dem vorgegebenen Referenzphasenwinkel sowie dem gemessenen Wechseldruck am Mund als Maß für die Gesamtimpedanz und dem gemessenen Gesamtphasenwinkel.

Vorteilhafte Weiterbildungen sind in den Unteransprüchen beschrieben. Die Erfindung eignet sich insbesondere bei der Verwendung des Gerätes "Siregnost FD 5", der Firma Siemens.

Ein Ausführungsbeispiel der Erfindung wird nachstehend anhand' der beigefügten Zeichnungen im einzelnen erläutert. In den Zeichnungen zeigt

Fig. 1 eine schematische Darstellung der vorbekannten Vorrichtung zur Bestimmung des Atemwegswiderstandes eines Patienten nach der Methode der oszillatorischen Widerstandsbestimmung nach dem Oberbegriff des Anspruchs 1, nämlich des Gerätes "Siregnost FD 5" der Firma Siemens und

Fig. 2 eine schematische Darstellung des Ersatzsystems der erfindungsgemäßen Vorrichtung.

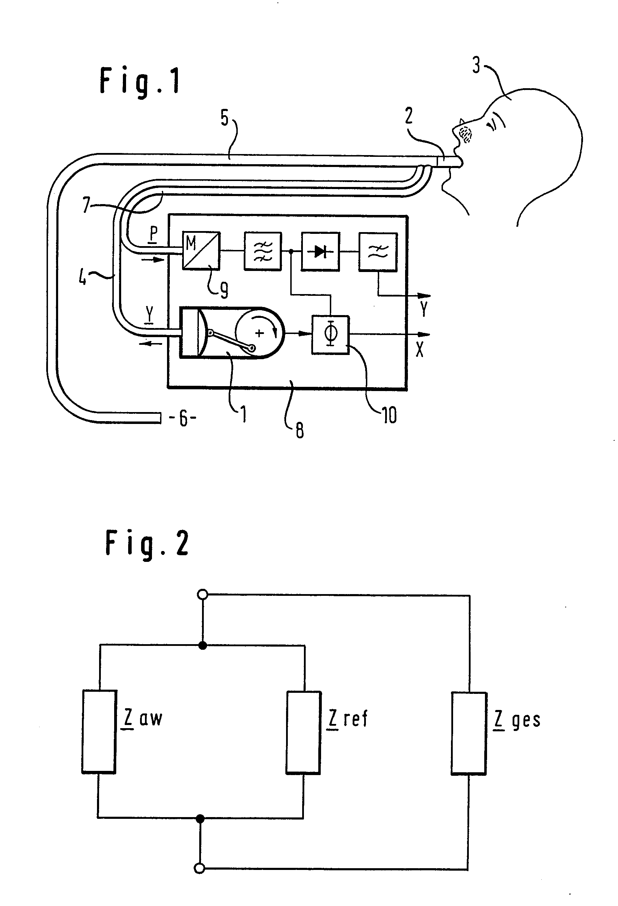

Die in Fig. 1 dargestellte Vorrichtung nach dem Oberbegriff des Anspruchs 1, die dem vorbekannten Gerät "Siregnost FD 5" der Firma Siemens entspricht, besitzt eine ventillose Membranpumpe 1 zum Erzeugen einer Luftschwingung mit einer vorbestimmten Amplitude und Frequenz, ein Luftschlauch-Mundstück (2) für den Patienten (3), einen Verbindungsschlauch (4) von der Pumpe (1) zum Mundstück (2), einen geeichten Luftschlauch (5) mit einer vorbestimmten Referenzimpedanz Zref vom Mundstück (2) ins Freie (6), einen Meßschlauch (7) vom Mundstück (2) zur Vorrichtung (8), einen Druckaufnehmer (9), der aus einem Mikrophon besteht, am Ende des Meßschlauches (7) zum Messen des Luftdrucks dp und zum Erzeugen eines Wechseldrucksignals, das aus einem analogen Spannungssignal besteht und aus einem Phasenmeßglied, das ein dem Phasenwinkel zwischen Volumenstrom und Wechseldruck des Gesamtsystems (Gesamtimpedanz) analoges Spannungssignal liefert. Im übrigen wurde das vorbekannte Gerät bereits in der Anleitung beschrieben. Weiterhin ist eine in der Fig. 1 nicht gezeichnete Recheneinrichtung vorhanden, die geeignet ist, die Atemtraktimpedanz $\underline{Z}aw$ aus der Referenzimpedanz $\underline{Z}ref$, der Gesamtimpedanz $\underline{Z}ges$ sowie dem Phasenwinkel $\Phi$ des Gesamtsystems zu berechnen.

3

Die Fig. 2 zeigt ein Ersatzsystem, bestehend aus der Atemtraktimpedanz Zaw, der Referenzimpedanz Zref und der Gesamtimpedanz Zges. Wie aus der Fig. 2 ersichtlich, resultiert die Gesamtimpedanz Zges aus einer Parallelschaltung der Atemtraktimpedanz Zaw und der Referenzimpedanz Zref.

Der geeichte Luftschlauch (5) führt vom Mundstück (2) des Patienten (3) ins Freie (6), damit der Patient (3) ungehindert atmen kann. Der geeichte Luftschlauch stellt den Referenzwiderstand Zref dar. Von der Pumpe (1) führt der Verbindungsschlauch (4) zum geeichten Luftschlauch (5).

Die Verbindungsstelle des Verbindungsschlauchs 4 mit dem geeichten Luftschlauch 5 liegt im Mundstück 2, also in der Nähe des Mundes des Patienten 3. Die von der Pumpe 1 über den Verbindungsschlauch 4 an den geeichten Luftschlauch 5 gelieferten Oszillationen setzen sich zum einen im geeichten Luftschlauch 5 fort, zum anderen im Atemsystem des Patienten 3. Hieraus folgt, daß der geeichte Luftschlauch und damit die Referenzimpedanz Zref einerseits und das Atemsystem des Patienten 3 und damit die Atemwegsimpedanz Zaw andererseits parallel geschaltet sind, wie in Fig. 2 gezeigt.

Ebenfalls im Mundstück 2 befindet sich die Anschlußstelle des Meßschlauches 7, der zum Druckaufnehmer 9 führt. Der Druckaufnehmer 9 wird durch ein Mikrophon gebildet. Das Phasenmeßglied 10 liefert als analoges Signal x den Phasenwinkel der Gesamtimpedanz Zges des Gesamtsystems, wobei das Gesamtsystem aus der Parallelschaltung der Referenzimpedanz Zref des geeichten Luftschlauchs 5 und der Atemwegsimpedanz Zaw des Atemweges des Patienten 3 besteht. Weiterhin liefert der Druckaufnehmer nach entsprechender Kalibrierung als analoges Signal y den Betrag der Gesamtimpedanz Zges des Gesamtsystems.

Die ventillose Membranpumpe 1 arbeitet mit einer Frequenz von 10 Hz und mit einem Hubvolumen von 1,4 ml. Der Oszillationsstrom der Pumpe 1 wird der menschlichen Atemfrequenz von 0,25 - 0,3 Hz aufgelagert. Der geeichte Luftschlauch 5 besteht aus einem am Mundstück 2 angeschlossenen Plastikschlauch mit vorgegebenem, bekanntem Radius und vorgegebener, bekannter Länge. Der geeichte Luftschlauch 5 dient als Referenzimpedanz Zref, dessen Wert von 10 mbar/l x s überwiegend durch die Induktivität der oszillierten Luftsäule verursacht wird.

Bei einem Phasenwinkel von 81° errechnet sich dabei als Realteil der Referenzimpedanz Zref 1,5 mbar/l x s. Vom Hersteller wurde bei einer stationären Strömung von 0,5 l/s ein Strömungswiderstand von 1,5 mbar/l x s im geeichten Luftschlauch (Referenz schlauch) 5 gemessen. Über den geeichten Luftschlauch 5 kann der Patient fast ungehindert atmen, sein Volumen von 85 ml vergrößert den Totraum nur unwesentlich.

Der oszillierende Volumenstrom erzeugt im Mund des Patienten einen Wechseldruck, der über den Meßschlauch 7 durch den Druckaufnehmer 9, also das Mikrophon, aufgenommen wird, das nach Filterung, Gleichrichtung und Glättung ein analoges Spannungssignal liefert.

Sämtliche Impedanzen, also sowohl die Atemwegsimpedanz Zaw als auch die Referenzimpedanz Zref als auch die Gesamtimpedanz Zges, sind komplexe Größen, also im mathematischen Sinn komplexe Zahlen mit einem Realteil und einem Imaginärteil. Für die oszillatorische Widerstandsbestimmung wird die in Fig. 1 gezeigte Anordnung, deren Ersatzsystem in der Fig. 2 dargestellt ist, als Analogon eines Wechselstromkreises betrachtet, in welchem zwei Widerstände - induktiver Referenzwiderstand Zref und Atemwiderstand Zaw - parallel geschaltet sind (Fig. 2). Die dabei zugrundeliegende Analogie elektrischer und pneumatischer Größen ist in folgender Tabelle wiedergegeben:

| Elektrotechnik | Pneumatik |
|---|---|
| Ladung Q | Volumen V |
| Spannung U | Druck p |
| Strom I = dQ/dt | Volumenstrom dV/dt |
| Widerstand R = U/I | Strömungswiderstand R = p/(dV/dt) |
| Kapazität C = dQ/dU | Kapazität C = dV/dp |
| Induktivität L = - U/(d2Q/dt2) | Induktivität L = - p/ (d2V/dt2) |

Bei Parallelschaltung von Wechselstromwiderständen, also Impedanzen, gelten die Regeln für Gleichstromwiderstände, also auch das zweite Kirchhoff'sche Gesetz :

1/Zges = 1/Zref + 1/Zaw    (1) (Unterstrich als Symbol für komplexe Größe)

Die Gesamtimpedanz Zges ist also durch die Referenzimpedanz Zref und den Atemwiderstand Zaw festgelegt. Die Referenzimpedanz Zref ist durch den geeichten Luftschlauch 5 vorgegeben. Die Gesamtimpedanz Zges kann durch den Druckaufnehmer 9 gemessen werden, dadV/dt konstant ist. Aus der vorgegebenen Referenzimpedanz Zref und der gemessenen Gesamtimpedanz Zges kann also nach der oben angegebenen Formel die Atemtraktimpedanz $\underline{Z}aw$ berechnet werden. Dies geschieht in der erfindungsgemäßen Recheneinrichtung nach der oben angegebenen Formel. Zu beachten ist, daß sämtliche Impedanzen ($\underline{Z}ges$, $\underline{Z}ref$, $\underline{Z}aw$) komplexe (also im mathematischen Sinn komplexe) Größen sind.

Beachtet man, daß die Gesamtimpedanz $\underline{Z}ges$ durch die Formel

$$\underline{Z}ges = dp/(dV/dt)$$

gegeben ist, folgt aus der oben angegebenen Formel (1):

$$\underline{Z}aw = (\underline{Z}ref \times dp)/((dV/dt) \times \underline{Z}ref - dp) \qquad (2)$$

Auch hier sind sämtliche Werte komplexe Größen, insbesondere also auch der Wechseldruck dp im Mund des Patienten und der kon stante Volumenstrom dV/dt. Die Formel (2) läßt sich auch wie folgt umformen:

$$\underline{Z}aw = 1/((dV/dt)/dp - 1/\underline{Z}ref) \qquad (3)$$

Dabei sind $\underline{Z}ref$ als induktiver Referenzwiderstand sowie dV/dt als Hubvolumen der Membranpumpe 1 pro Zeit bekannt. dp kann leicht durch den Druckaufnehmer 9 bzw. das Mikrophon gemessen werden. Es läßt sich somit allein aus dem gemessenen Oszillationsdruck dp auf den (komplexen) Atemwiderstand $\underline{Z}aw$ rückschließen. Auch in der Gleichung (3) sind nur komplexe Größen enthalten.

Da in dem in Fig. 2 gezeigten, analogen Ersatzschaltkreis der oszillatorischen Widerstandsmessung sowohl die Einzelwiderstände (Einzelimpedanzen) $\underline{Z}ref$ und $\underline{Z}aw$ als auch der Gesamtwiderstand (Gesamtimpedanz) $\underline{Z}ges$ komplexe Größen im Sinne einer Impedanz darstellen, sind sie jeweils durch eine Phasenverschiebung zwischen dp und dV/dt gekennzeichnet. Mit der in Fig. 1 dargestellten, vorbekannten Vorrichtung (Siregnost FD 5) läßt sich die Phasendifferenz zwischen dV/dt und dp des Gesamtsystems (geeichter Luftschlauch 5 und Atemtrakt des Patienten 3) messen. Der rotierende Teil der Pumpe 1 durchläuft hierzu eine Lichtschranke, welche die Stellung der Pumpe und damit die Phase von dV/dt abtastet. Mit einem Phasenmeßglied (10) wird die Phasendifferenz zum gemessenen Wechseldruck dp (vom Meßaufnehmer 9 gemessen) bestimmt und als Signal x abgegeben. Als Signal y wird der Betrag der Impedanz $\underline{Z}ges$ des Gesamtsystems (geeichter Luftschlauch 5 und Atemtrakt des Patienten 3) abgegeben.

Die erfindungsgemäße Recheneinrichtung ist vorzugsweise geeignet, die Atemwegsimpedanz $\underline{Z}aw$ aus der Referenzimpedanz $\underline{Z}ref$ und der Gesamtimpedanz $\underline{Z}ges$ nach folgender Formel zu berechnen:

$$\underline{Z}aw = (\underline{Z}ges \times \underline{Z}ref)/(\underline{Z}ref - \underline{Z}ges) \qquad (4)$$

Die Formel (4) ergibt sich durch Auflösung der Formel (1) nach $\underline{Z}aw$.

Durch Einsetzen von $\underline{Z}ges = dp/(dV/dt)$ in (4) ergibt sich die o. a. Formel (2). Kürzt man die Formel (2) um $\underline{Z}ref \times dp$, so ergibt sich die o. a. Formel (3). Sowohl die Gesamtimpedanz $\underline{Z}ges$ als auch die Atemtraktimpedanz $\underline{Z}aw$ und die Referenzimpedanz $\underline{Z}ref$ sind durch Phasenwinkel zwischen Volumenstrom dV/dt und Wechseldruck dp gekennzeichnet. Ist f der Phasenwinkel der Atemtraktimpedanz und $\Phi$ der Phasenwinkel der Gesamtimpedanz und $\epsilon$ der Phasenwinkel der Referenzimpedanz, so läßt sich damit die Gleichung (3) in komplexer Schreibweise wie folgt schreiben:

$$\underline{Z}aw * e^{jf} = \frac{1}{((d\underline{V}/dt)/dp) * e^{-j\Phi} - (1/\underline{Z}ref) * e^{-j\epsilon}} \qquad (5)$$

Durch Anwendung der Rechenvorschriften für komplexe Zahlen kann daraus der reale Atemwiderstand Raw und der Phasenwinkel f als Kenngröße des Imaginärteils der Atemtraktimpedant berechnet werden.

Vorzugsweise ist ein Analog-Digital-Wandler vorgesehen, der die analog gemessene Gesamtimpedanz $\underline{Z}ges$ und den Gesamtphasenwinkel $\Phi$ digitalisiert und der Recheneinrichtung kontinuierlich zuführt. Zur Berechnung der Atemtraktimpedanz, des realen Atemtraktwiderstandes und des Phasenwinkels des Atemtraktes muß auch die Referenzimpedanz $\underline{Z}ref$ und der Phasenwinkel der Referenzimpedanz $\epsilon$ in digitalisierter Form der Recheneinrichtung eingegeben werden. Dies macht keine Schwierigkeiten, da die Referenzimpedanz $\underline{Z}ref$ und der Referenzimpedanz-Phasenwinkel $\epsilon$ vorgegeben und daher vorbekannt sind. Die Recheneinrichtung beinhaltet in diesem Fall vorzugsweise einen Mikroprozessor, der die digitalisierten Signale verarbeitet, vorzugsweise nach einer der o. g. Formeln mit den Rechenvorschriften für komplexe Zahlen.

Die Recheneinrichtung ist geeignet, aus dem komplexen Atemtraktwiderstand $\underline{Z}aw$ den realen Atemtraktwiderstand $\underline{Z}aw$ und den Phasenwinkel f des Atemtraktes zu berechnen. Der reale Atemtraktwiderstand Raw ist der Realteil des komplexen Atemtraktwiderstandes $\underline{Z}aw$. Der Phasenwinkel f des Atemtraktes ist der Phasenwinkel des komplexen Atemtraktwiderstandes $\underline{Z}aw$.

Vorzugsweise ist eine Anzeigeeinrichtung zur Anzeige der von der Recheneinrichtung berechneten Werte und/oder ein Drucker zum Ausdrucken der von der Recheneinrichtung berechneten Werte und/oder eine Aufzeichnungseinrichtung zum Aufzeichnen der von der Recheneinrichtung berechneten Werte vorhanden. Die Aufzeichnungseinrichtung kann insbesondere aus einem Oszillographen oder einem Schreiber bestehen. Weiterhin ist die Aufzeichnungseinrichtung vorzugsweise geeignet, die von der Recheneinrichtung berechneten Werte kontinuierlich aufzuzeichnen.

Die Recheneinrichtung ist vorzugsweise geeignet, kontinuierlich mit einer vorgegebenen Frequenz die Atemwegsimpedanz Zaw oder die anderen o. a. Werte zu berechnen. Die Recheneinrichtung wird also vorzugsweise kontinuierlich mit Eingangswerten versorgt, so daß sie kontinuierlich die Ausgangswerte berechnet. Weiterhin ist die Recheneinrichtung vorzugsweise geeignet, aus einer beliebigen Anzahl von Meßwerten einen Mittelwert zu bilden.

Die erfindungsgemäße Vorrichtung bringt mehrere Vorteile mit sich. Insbesondere ist diese Vorrichtung genauer als vorbekannte Vorrichtungen. Die Gesamtimpedanz Zges sowie der reale Atemtraktwiderstand Raw und der Atemtraktphasenwinkel f, die gemessen werden, sollten zwar zeitlich konstant sein, in der Praxis zeigt es sich jedoch, daß bei nahezu jedem Patienten diese Gesamtimpedanz Zges in zeitlicher Hinsicht Schwankungen unterworfen ist. Im allgemeinen schwankt die Gesamtimpedanz Zges atemsynchron. Es ist daher vorteilhaft, einen Mittelwert zu bilden; diese Mittelwertbildung ist nach der Erfindung einfach, schnell, exakt und wiederholt möglich. Aus den gemessenen und berechneten Werten kann ein exakter Mittelwert errechnet werden.

Weiterhin kann durch die mit vorgegebener Frequenz wiederholte fortlaufende Berechnung eine kontinuierliche Aufzeichnung realisiert werden, beispielsweise durch einen Oszillographen, einen Schreiber oder ähnliches. Dies ist insbesondere in der Intensivmedizin, in der Narkoseüberwachung, bei der Überprüfung der Wirksamkeit von Pharmaka und/oder in der Allergologie bei Provokationstests wünschenswert bzw. erforderlich.

Als weiterer Vorteil kommt hinzu, daß eine Mitarbeit (Kooperation) des Patienten nicht erforderlich ist. Der Patient wird auch nicht beeinträchtigt; die Vorrichtung kann auch bei bewußtlosen Patienten benutzt werden.

**Ansprüche**

1. Vorrichtung zur Bestimmung des Atemtraktwiderstandes eines Patienten nach der Methode der oszillatorischen Widerstandsbestimmung, bestehend aus
einer Pumpe (1) , vorzugsweise einer ventillosen Membranpumpe, zum Erzeugen einer Luftschwingung mit einer vorbestimmten Amplitude und Frequenz,
einem Luftschlauch-Mundstück (2) für den Patienten (3),
einem Verbindungsschlauch (4) von der Pumpe (1) zum Mundstück (2),
einem geeichten Luftschlauch (5) mit einer vorbestimmten Referenzimpedanz (Zref) vom Mundstück (2) ins Freie (6),
einem Meßschlauch (7) vom Mundstück (2) zur Vorrichtung (8),
einem Druckaufnehmer (9) , vorzugsweise einem Mikrophon, in dem Meßschlauch (7) zum Messen des Luftdrucks (dp) und zum Erzeugen eines Luftdrucksignals, vorzugsweise eines analogen Spannungssignals als Maß für die Gesamtimpedanz Zges, wobei das Gesamtsystem aus der Referenzimpedanz (Zref) des geeichten Luftschlauchs (5) und der Atemtraktimpedanz (Zaw) des Atemweges des Patienten (3) besteht, und einem Phasenmeßglied (10) zum Messen der Phasendifferenz zwischen der Phase des Volumenstroms (dV/dt) und dem Wechseldruck am Mund (dp) des Gesamtsystems und zum Erzeugen eines Phasendifferenzsignals (Gesamtimpedanz) , vorzugsweise eines analogen Spannungssignals,

**gekennzeichnet durch**
eine Recheneinrichtung, die geeignet ist, die Atemtraktimpedanz (Zaw) aus der Referenzimpedanz (Zref) und der Gesamtimpedanz (Zges) zu berechnen.

2. Vorrichtung nach Anspruch 1, dadurch gekennzeichnet, daß die Recheneinrichtung geeignet ist, die Atemtraktimpedanz (Zaw) aus der Referenzimpedanz (Zref) und der Gesamtimpedanz (Zges) nach der Formel
$$Zaw = (Zges \times Zref)/(Zref - Zges)$$
zu berechnen.

3. Vorrichtung nach Anspruch 1, dadurch gekennzeichnet, daß die Recheneinrichtung geeignet ist, die Atemtraktimpedanz (Zaw) aus der Referenzimpedanz (Zref) und der Gesamtimpedanz (Zges) nach der Formel
$$Zaw = 1/((dV/dt)/dp - 1/Zref)$$

zu berechnen, wobei dV/dt der über die Zeit gemittelte Volumenstrom der Pumpe (1) ist.

4. Vorrichtung nach einem der vorhergehenden Ansprüche, gekennzeichnet durch einen Analog-Digital-Wandler, der die analog gemessene Gesamtimpedanz ($\underline{Z}$ges) sowie den Phasendifferenzwinkel f der Gesamtimpedanz ($\underline{Z}$ges) digitalisiert und der Recheneinrichtung zuführt.

5. Vorrichtung nach Anspruch 4, dadurch gekennzeichnet, daß die Recheneinrichtung einen Mikroprozessor beinhaltet.

6. Vorrichtung nach einem der vorhergehenden Ansprüche, dadurch gekennzeichnet, daß die Recheneinrichtung geeignet ist, aus der komplexen Gesamtimpedanz ($\underline{Z}$ges) und dem Gesamtphasenwinkel $\Phi$ den reellen Atemtraktwiderstand (Raw) zu berechnen.

7. Vorrichtung nach einem der vorhergehenden Ansprüche, dadurch gekennzeichnet, daß die Recheneinrichtung geeignet ist, aus der komplexen Gesamtimpedanz ($\underline{Z}$ges) und dem Phasenwinkel des Gesamtsystems $\Phi$ den Phasenwinkel f des Atemtrakts zu berechnen.

8. Vorrichtung nach einem der vorhergehenden Ansprüche, gekennzeichnet durch eine Anzeigeeinrichtung zur Anzeige der von der Recheneinrichtung berechneten Werte und/oder einem Drucker zum Ausdrucken der von der Recheneinrichtung berechneten Werte und/oder eine Aufzeichnungseinrichtung, insbesondere einen Oszillograph oder einen Schreiber, zum Aufzeichnen, vorzugsweise zum kontinuierlichen Aufzeichnen der von der Recheneinrichtung berechneten Werte.

9. Vorrichtung nach einem der vorhergehenden Ansprüche, dadurch gekennzeichnet, daß die Recheneinrichtung geeignet ist, aus einer beliebigen Anzahl von berechneten Werten einen Mittelwert zu bilden.

# Fig.1

# Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-B-2714216 (SIEMENS A.G.)<br>* Spalte 4, Zeile 9 - Spalte 6, Zeile 64;<br>Ansprüche 1, 3, 5, 6; Figuren *<br>--- | 1-3, 6,<br>7 | A61B5/085<br>G01R27/00 |
| Y<br><br>A | DE-A-2631470 (SIEMENS A.G.)<br>* Seite 5, Zeile 22 - Seite 8, Zeile 34 *<br>* Ansprüche 1, 4, 14; Figuren *<br>--- | 1, 4, 5,<br>8<br>6, 7 | |
| Y<br><br>A | DE-A-3521633 (PHILIPS PATENTVERWALTUNG G.M.B.H.)<br>* Seite 3, Zeile 13 - Seite 4, Zeile 6 *<br>* Seite 4, Zeile 33 - Seite 6, Zeile 16 *<br>* Figuren *<br>--- | 1, 4, 5,<br>8<br>2 | |
| A | US-A-4031885 (J.E.P. DAVIS U.A.)<br>* Spalte 2, Zeile 60 - Spalte 3, Zeile 18;<br>Figuren 1, 2 *<br>* Spalte 4, Zeile 4 - Spalte 5, Zeile 9 *<br>--- | 1, 3-5,<br>8 | |
| A | ELECTRO MEDICA (SIEMENS).<br>vol. 47, no. 4, 1979, ERLANGEN DE<br>Seiten 149 - 156; I. Brambilla u.a.:<br>"Der oszillatorische Atemwiderstand (Ros) in der<br>Schwangerschaft und im Wochenbett"<br>* Seiten 150 - 151 *"Verfahren"; Fig. 2<br>------ | 1 | **RECHERCHIERTE SACHGEBIETE (Int. Cl.5 )**<br><br>A61B<br>G01R |

Der vorliegende Recherchenbericht wurde für alle Patentansprüche erstellt

| Recherchenort | Abschlußdatum der Recherche | Prüfer |
|---|---|---|
| DEN HAAG | 21 MAERZ 1990 | RIEB K.D. |